(19) Europäisches Patentamt
European Patent Office
Office européen des brevets

(11) Publication number: **0 010 553**
**B1**

(12) # EUROPEAN PATENT SPECIFICATION

(45) Date of publication of patent specification: **18.05.83**

(51) Int. Cl.$^3$: **C 07 D 207/337**

(21) Application number: **78101245.5**

(22) Date of filing: **27.10.78**

(54) **Process of reducing a loweralkyl 1-loweralkyl-pyrrole-2-glyoxylate to a loweralkyl 1-loweralkyl-pyrrole-2-acetate.**

(43) Date of publication of application:
**14.05.80 Bulletin 80/10**

(45) Publication of the grant of the patent:
**18.05.83 Bulletin 83/20**

(84) Designated Contracting States:
**BE CH DE FR GB LU NL SE**

(56) References cited:
DE - A - 2 737 946
DE - B - 1 301 313
US - A - 3 752 826
US - A - 3 803 169
US - A - 3 846 447
US - A - 3 957 818

JOURNAL FÜR PRAKTISCHE CHEMIE, vol. 35, 1967, Leipzig, M. SCHEITHAUER et al. "Zur Einwirkung von Schwefelwasserstoff auf 1,2-Diketone, alpha-Oxo-carbonsäureester und Carbonsäurenhydride bei Raumtemperatur" pages 41 to 48

**The file contains technical information submitted after the application was filed and not included in this specification**

(73) Proprietor: **McNeilab, Inc.**
**Camp Hill Road**
**Fort Washington Pennsylvania 19034 (US)**

(72) Inventor: **Carmosin, Richard**
**1241, 65th Avenue**
**Philadelphia, Pa. (US)**

(74) Representative: **Vossius Vossius Tauchner Heunemann Rauh et al,**
**Siebertstrasse 4 P.O. Box 86 07 67**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 010 553 B1

# Process of reducing a loweralkyl 1-loweralkyl-pyrrole-2-glyoxylate to a loweralkyl 1-loweralkyl-pyrrole-2-acetate

In J. Prakt.Chem., Vol. 35, p. 41 (1967), the base-catalyzed action of hydrogen sulfide on certain 1,2-diketones and certain glyoxylic acid esters are reported.

It is an object of the invention to provide a process for producing loweralkyl 1-loweralkyl-pyrrole-2-acetates from loweralkyl 1-loweralkyl-pyrrole-2-glyoxylates whereby a marked increase in percent conversion from glyoxylate precursor to acetate product is obtained.

The end products of the process of the subject invention are 1-loweralkyl-pyrrole-2-acetates (I) which have been reported in the literature as being useful intermediates in the preparation of 5-aroyl-pyrrole-2-acetic acid derivatives having anti-inflammatory activity (e.g., see U.S. Patent Nos. 3 752 826, 3 803 169, 3 846 447 and 3 957 818).

This invention relates to an improved process of preparing loweralkyl 1-loweralkyl-pyrrole-2-acetates of the general formula (I)

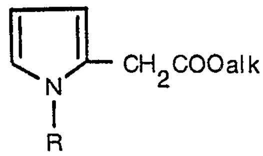

(I)

wherein R is loweralkyl, preferably methyl; and alk is loweralkyl, preferably methyl.

In accordance with this invention, there is provided an improved process of reducing a loweralkyl 1-loweralkyl-pyrrole-2-glyoxylate to a loweralkyl 1-loweralkyl-pyrrole-2-acetate by the amine-catalyzed action of hydrogen sulfide in a tertiary amine solvent or a dipolar aprotic organic solvent, the improvement comprising the reduction being conducted under a pressure of at least (2.11 kg/cm$^2$) 205.92 KPa. Accordingly, a marked increase in percent conversion from glyoxylate precursor to acetate product is obtained.

As used herein, "loweralkyl" refers to straight or branch chained alkyls having from 1 to 6 carbons, for example, methyl, ethyl, propyl, isopropyl, butyl, tert.-butyl, pentyl and hexyl.

According to the instant process, an appropriate loweralkyl 1-loweralkyl-pyrrole-2-glyoxylate of formula (II), wherein R and alk are as previously defined, is reduced to the corresponding loweralkyl 1-loweralkyl-pyrrole-2-acetate of formula (I) by the base-catalyzed action of hydrogen sulfide under pressure:

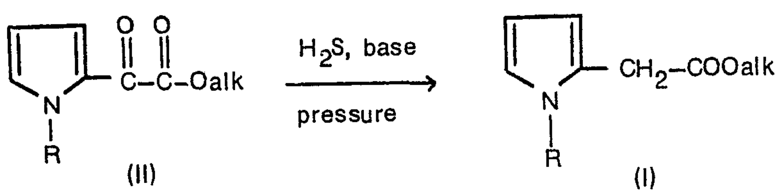


(II) (I)

Base catalysts suitable for the foregoing reduction reaction are tertiary amines, for example, a tertiary alkyl amine, such as triethylamine and diisopropylethylamine; a heterocyclic amine, e.g., N-methyl-pyrrolidine, N-methyl-piperidine and N-methyl-piperazine; a heteroaryl amine, e.g., pyridine, 2-hydroxy-pyridine, imidazole, N-methyl-imidazole and bipyridyl; and triethylenediamine. The most preferred amines are pyridine, imidazole and N-methylpiperidine.

Base catalysts which are liquids and also suitable as solvents for the reduction reaction, e.g., pyridine and triethylamine, may be employed without the use of additional solvent. Other solvents that may be utilized for the reduction reaction, although less preferred, are dipolar aprotic organic solvents, such as, for example, N,N-dimethylformamide (DMF), dimethyl sulfoxide (DMSO) and hexamethylphosphoramide (HMPA).

The reaction temperature is not critical and temperatures varying from ambient temperature to 120°C may be conveniently utilized, although elevated temperatures are advantageously employed to enhance the rate of reaction and temperatures varying from 60°C to 100°C are preferred.

The reduction reaction is run in an appropriate sealed vessel capable of withstanding an internal pressure of at least (2.11 kg/cm$^2$) 205.92 kPa of gaseous hydrogen sulfide. The preferred pressure range is from (3.52 to 35.2 kg/cm$^2$) 345.19 to 3451.9 kPa although pressures up to (70.3 kg/cm$^3$) 6894.08 kPa may be employed.

The following examples are provided for purposes of illustrating the subject invention but not for limiting same thereto.

## Example I

A solution of 50g of methyl 1-methylpyrrole-2-glyoxylate in 200 ml of pyridine is cooled to

$-78°C$ and 64g of hydrogen sulfide is added. The mixture is sealed in a stirred autoclave and heated to 63°C. The pressure rises to about (9.14 $kg/cm^2$) 896.33 kPa. After 27 hrs., the hydrogen sulfide is driven off in a nitrogen stream and the solution is decanted from the precipitated sulfur. Pyridine is distilled off at (20 mm Hg) 2.67 kPa. The residue is distilled at (0.03 mm Hg) 0.004 kPa, b.p. 68—70°C, to give 39.2g of oily methyl 1-methylpyrrole-2-acetate (86% yield).

Example II

A solution of 25g of methyl 1-methylpyrrole-2-glyoxylate, 23g of hydrogen sulfide and 100 ml pyridine is sealed in an autoclave and stirred 46 hrs. at 25°C under pressure of (4.22 $kg/cm^2$) 411.84 kPa. The pressure is released and the solution decanted from precipitated sulfur. The solution is taken up in diethyl ether and washed successively with potassium carbonate solution, dilute hydrochloric acid, sodium bicarbonate solution and brine. The solution is then dried over $MgSO_4$ and the solvent evaporated in vacuo. The residual oil is distilled at (0.001 to 0.005 mmHg) 0.00013 to 0.00075 kPa to give 18.8g of oily methyl 1-methylpyrrole-2-acetate (82% yield).

Example III

A solution of 44g (0.26 mole) of methyl N-methyl-2-pyrrole-2-glyoxylate in 110g (1.4 mole) of pyridine is placed in a stirred autoclave. The solution is pressurized with hydrogen sulfide gas, with stirring, until the pressure is stabilized at about (14.1 $kg/cm^2$) 1382.8 kPa. Stirring is continued at room temperature for four hours. The pressurized vessel is opened and the contents assayed by gas liquid chromatography (GLC) indicating 33% of the product, methyl 1-methylpyrrole-2-acetate.

Example IV

A mixture of 1g of the loweralkyl 1-methylpyrrole-2-glyoxylate and quantities of the solvent and catalyst, shown in the following Table, hereafter are cooled to $-60°C$ and 1.8g hydrogen sulfide is added. Each of the mixtures is sealed in a pressure bottle and warmed to the indicated temperature. After the specified reaction time, the mixture is diluted with chloroform and analyzed by gas liquid chromatography against triphenylmethane internal standard (OV 17; 90—280°C) to yield the indicated percent conversion for each of the respective loweralkyl 1-methylpyrrole-2-acetates obtained as corresponding products.

| Alkyl | Catalyst | Solvent | Time (hrs.) | Temp. (°C.) | % Conversion |
|---|---|---|---|---|---|
| $CH_3$ | pyridine, 2.6 ml | (pyridine) | 4 | 25 | 27 |
| $CH_3$ | imidazole, .224g | pyridine, 2.6 ml | 4 | 25 | 39 |
| $CH_3$ | N-methylimidazole; .27g | DMSO, 10.6 ml | 4 | 25 | 16 |
| $CH_3$ | imidazole 2.24g | DMSO, 10.6 ml | 4 | 25 | 29 |
| $CH_3$ | 2-hydroxypyridine, 1.1g | pyridine, 4 ml | 4 | 25 | 33 |
| $C_2H_5$ | triethylenediamine, 0.34g | DMSO, 10.6 ml | 4 | 25 | 17 |
| $C_2H_5$ | 2,2-bipyridyl, 4.75g | DMSO, 20.6 ml | 4 | 25 | 6.9 |
| $C_2H_5$ | pyridine, 2.6 ml | DMSO, 10.6 ml | 4 | 25 | 15.6 |
| $C_2H_5$ | pyridine, 2.6 ml | (pyridine) | 4 | 25 | 23 |
| $C_2H_5$ | triethylamine, 4.2 ml | (triethylamine) | 4 | 25 | 7.5 |
| $C_2H_5$ | imidazole, 0.9g | pyridine, 4 ml | 4 | 77 | 91 |
| $CH_3$ | N-methylpyrrolidine, 3.4 ml | (N-methylpyrrolidine) | 22 | 25 | 55 |
| $CH_3$ | diisopropyethylamine, 4.2 ml | DMSO, 5.3 ml | 69 | 25 | 9 |
| $CH_3$ | N-methylpiperazine, 3.65 ml | (N-methylpiperazine) | 27 | 25 | 14.6 |

Example V

A. A solution of 1.0 g of methyl 1-methylpyrrole-2-glyoxylate in 2.66 ml pyridine and 10.6 ml of DMSO is cooled to $-60°C$ and 1.8 g hydrogen sulfide is added. The reaction mixture is sealed in a pressure

bottle and stirred for 4 hrs. at room temperature. The pressure is then released and the reaction mixture diluted with chloroform and analyzed by gas liquid chromatography. A 23% conversion to methyl 1-methyl-pyrrole-2-acetate is observed.

B. By following the procedure of Example V—A, but substituting an equivalent amount of DMF and HMPA for the DMSO solvent, the following respective conversions are found: 5.5% for DMF and 6.0 for HMPA.

Example VI (Comparative Example)

The experimental conditions of Sheithauer and Mayer [J. Prakt, Chem., Vol. 35, p. 47 (1967)] are employed utilizing ethyl 1-methylpyrrole-2-glyoxylate as the starting material for conversion to ethyl 1-methylpyrrole-2-acetate. Hydrogen sulfide is bubbled vigorously through a stirred solution of 1 g of ethyl 1-methylpyrrole-2-glyoxylate, 1 ml of pyridine and 9 ml of DMF for 4 hrs. at 25°C. The mixture is analyzed by gas liquid chromatography. A 1.1% conversion to ethyl 1-methylpyrrole-2-acetate is measured.

Example VII (Comparative Example)

A solution of methyl 1-methylpyrrole-2-glyoxylate is treated with hydrogen sulfide as in the preceding example. A 2.3% conversion to methyl 1-methylpyrrole-2-acetate is measured.

**Claims**

1. Process of reducing a loweralkyl 1-loweralkyl-pyrrole-2-glyoxylate to a loweralkyl 1-loweralkyl-pyrrole-2-acetate by the amine-catalyzed action of hydrogen sulfide in a tertiary amine solvent or a dipolar aprotic organic solvent, loweralkyl meaning straight or branch chained alkyls having 1 to 6 carbon atoms, characterized by carrying out said reduction under a pressure of at least (2.11 kg/cm$^2$) 205.92 kPa.

2. Process according to claim 1, characterized by carrying out said reduction under a pressure of from (3.52 to 35.2 kg/cm$^2$) 345.19 to 3451.9 kPa.

3. Process according to claim 2 for reducing methyl 1-methyl-pyrrole-2-glyoxylate to methyl 1-methyl-pyrrole-2-acetate.

4. Process according to claim 3 characterized by carrying out the reduction in the presence of pyridine.

**Revendications**

1. Procédé pour réduire un 1-alkyl inférieur-pyrrole-2-glyoxylate d'alkyle inférieur en un 1-alkyl inférieur-pyrrole-2-acétate d'alkyle inférieur par action catalysée par une amine de l'acide sulfhydrique dans un solvant constitué d'une amine tertiaire ou dans un solvant organique aprotique dipolaire, alkyle inférieur désignant des alkyles droits ou ramifiés ayant 1 à 6 atomes de carbone, caractérisé en ce qu'on effectue ladite réduction sous une pression d'au moins (2,11 kg/cm$^2$) 205,92 kPa.

2. Procédé selon la revendication 1, caractérisé en ce qu'on effectue ladite réduction sous une pression de (3,52 à 35,2 kg/cm$^2$) 345,19 à 3451,9 kPa.

3. Procédé selon la revendication 2 pour réduire le 1-méthyl-pyrrole-2-glyoxylate de méthyle en 1-méthyle-pyrrole-2-acétate de méthyle.

4. Procédé selon la revendication 3, caractérisé en ce qu'on effectue la réduction en présence de pyridine.

**Patentansprüche**

1. Verfahren zur Reduktion eines Niederalkyl-1-niederalkylpyrrol-2-glyoxalats in ein Niederalkyl-1-niederalkylpyrrol-2-acetat durch amin-katalysierte Reaktion von Schwefelwasserstoff in einem tert.-Amin-Lösungsmittel oder einem dipolaren aprotischen organischen Lösungsmittel, wobei niederalkyl geradkettige oder verzweigtkettige Akylreste mit 1 bis 6 Kohlenstoffatomen bedeutet, dadurch gekennzeichnet, daß man die Reduktion unter einem Druck von mindestens (2,11 kg/cm$^2$) 205,92 kPa durchführt.

2. Verfahren nach Anspruch 1, dadurch gekennzeichnet, daß man die Reduktion unter einem Druck von (3,52 bis 35,2 kg/cm$^2$) 345,19 bis 3451,9 kPa durchführt.

3. Verfahren nach Anspruch 2, zur Reduktion von Methyl-1-methylpyrrol-2-glyoxalat in Methyl-1-methyl-pyrrol-2-acetat.

4. Verfahren nach Anspruch 3, dadurch gekennzeichnet, daß man die Reduktion in Gegenwart von Pyridin durchführt.